# EUROPÄISCHE PATENTSCHRIFT

(11) **EP 0 956 827 B1**
(45) Veröffentlichungstag und Bekanntmachung des Hinweises auf die Patenterteilung: **13.10.2004**
(21) Anmeldenummer: 99108284.3
(22) Anmeldetag: 27.04.1999
(51) Int. Cl.: A61B 18/00, A61B 18/14

(54) **Elektrochirurgisches Instrument**
Electrosurgical apparatus
Appareil électrochirurgical

(30) Priorität: 06.05.1998 DE 19820240
(43) Veröffentlichungstag der Anmeldung: 17.11.1999
(73) Patentinhaber: Erbe Elektromedizin GmbH., D-72072 Tübingen (DE)
(72) Erfinder: Farin, Günter, 72070 Tübingen (DE); Grund, Karl Ernst, Prof. Dr. med., 72070 Tübingen (DE)
(74) Vertreter: Bohnenberger, Johannes, Dr.

(56) Entgegenhaltungen:
- EP-A- 0 787 465
- WO-A-97/26034
- WO-A-98/01075
- DE-A- 3 710 489
- DE-A- 19 711 673

## Beschreibung

Die Erfindung betrifft ein elektrochirurgisches Instrument zur Koagulation biologischer Gewebe nach dem Oberbegriff des Patentanspruches 1.

Die Plasmachirurgie ist ein Verfahren der monopolaren Hochfrequenzchirurgie (HF-Chirurgie), bei welchem ein von einem Hochfrequenzgenerator (HF-Generator) generierter, hochfrequenter elektrischer Strom (HF-Strom) durch ein ionisiertes Edelgas (Plasma), beispielsweise Argon (Argon-Plasma), von einem elektrischen Pol innerhalb eines hierfür geeigneten chirurgischen Instruments auf das zu behandelnde Gewebe appliziert und von dort durch eine am Patienten applizierte sogenannte Neutralelektrooe zum HF-Generator zurückgeleitet wird (G. Farin et al.: Technology of Argon Plasma Coagulation with Particular Regard to Endoscopic Applications; Endoscopic Surgery and Allied Technologies, No. 1, vol. 2, Februar 1994, 71-77). Hierdurch wird sowohl endogen durch den HF-Strom als auch exogen durch die zum Gewebe relativ höhere Temperatur des Plasmas Wärme in dieses Gewebe eingebracht, welche dessen Temperatur erhöht. In Abhängigkeit von der Temperatur werden im Gewebe verschiedene thermische Effekte verursacht, welche von Chirurgen für verschiedene therapeutische Zwecke, wie beispielsweise zur Blutstillung beziehungsweise Hämostase und/oder zur thermischen Devitalisation oder Destruktion pathologischer Gewebe genutzt werden können (K.E. Grund et al.: Argon Plasma Coagulation in Flexible Endoscopy, Endoscopic Surgery and Allied Technologies, No. 1, vol. 2, Februar 1994, 42-46).

Eine wesentliche physikalische Voraussetzung der Plasmachirurgie ist ein Edelgas, wie beispielsweise das genannte Argon oder Helium, welches zwischen dem elektrischen Pol, welcher von einer Elektrode innerhalb des Instruments gebildet wird und dem zu behandelndem Gewebe vorhanden sein muß. Edelgase sind nämlich im Vergleich zu Sauerstoff und/oder Stickstoff beziehungsweise Luft mit relativ geringen elektrischen Feldstärken ionisierbar und reagieren mit dem Gewebe nicht chemisch. Folglich wird das Gewebe auch nicht karbonisiert oder gar vaporisiert.

Innerhalb der letzten fünf Jahre hat die Plasmachirurgie, insbesondere in der flexiblen Endoskopie, ein breites Indikationsspektrum gefunden (K.E. Grund: DMW), welches jedoch unterschiedliche Anforderungen an die Applikationstechnik und die hierfür erforderlichen Instrumente, HF-Generatoren und Gasquellen stellen.

Aus der US 5,207,675 ist ein elektrochirurgisches Instrument bekannt, das allerdings insofern problematisch ist, als die Elektrode des Instruments unbeabsichtigt in eine direkte Gewebeberührung gelangen kann. Infolge der relativ hohen HF-Spannung an der Elektrode besteht dabei die Gefahr, daß insbesondere dünnwandige Organes wie sie im Gastrointestinaltrakt sowie im Tracheobronchialsystcm in der Regel vorliegen, perforiert werden.

Aus der DE 41 39 029 A1 ist ein elektrochirurgisches Instrument bekannt, bei welchem die Elektrode derart innerhalb des Instruments angeordnet ist, daß sie bei bestimmungsgemäßer Anwendung das Gewebe nicht berührt. Ein Problem bei diesem Instrument besteht darin, daß das Edelgas aus einer oder mehreren Öffnungen am distalen Ende des Instruments in eine vorgegebene Richtung strömt. Folglich kann der HF-Strom auch nur im jeweiligen (ionisierten) Gasstrahl zum Gewebe fließen. Bei bei Behandlung großflächiger Läsionen ist dies insofern nachteilig, als das Instrument mehrmals über das zu behandelnde Gewebe bewegt werden muß, um den HF-Strom und damit auch die beabsichtigten thermischen Effekte möglichst gleichmäßig über die gesamte großflächige Läsion zu applizieren.

Aus der DE 195 35 811 C1 ist ein elektrochirurgisches Instrument der eingangs genannten Art bekannt, bei welchem an der Gas-Ausströmöffnung ein Diffusor vorgesehen ist, der dazu dienen soll, eine mechanische Wirkung auf Flüssigkeit und Gewebe zu vermeiden. Insbesondere bei der Behandlung großflächiger Läsionen hat es sich aber gezeigt, daß infolge der unvermeidlichen oder sogar gewollten Turbulenzen der Strömung des Edelgases eine Vermischung des Edelgases mit dem Gas der Umgebung (in der Regel Luft) auftritt, wodurch die spezifischen Vorteile der Plasmachirurgie teilweise wieder aufgehoben werden.

Es ist Aufgabe der Erfindung, ein chirurgisches Instrument der eingangs genannten Art dahingehend weiterzubilden, daß in einfacher Weise auch großflächige Läsionen besser behandelbar sind.

Diese Aufgabe wird durch die im Patentanspruch 1 angegebenen Merkmale gelöst.

Ein wesentlicher Punkt der Erfindung liegt darin, daß einerseits ein fächerförmiger Strahl von Edelgas geformt und andererseits dieser Strahl so "strukturiert" wird, daß eine Vermischung mit umgebender Luft praktisch nicht auftritt. Gleichzeitig wird der Gasdruck hierbei derart geregelt, daß ein Fortblasen von Flüssigkeit vom Gewebe oder gar ein Eindringen von Gas in ein offenes Blutgefäß vermieden werden. Durch diese Maßnahmen ist gewährleistet, daß großflächige Läsionen schnell "abgefahren" werden können und dabei einerseits ein relativ geringer Gasstrom genügt, andererseits dennoch sicher die Gefahr der obengenannten Gewebezerstörung vermieden wird.

Vorzugsweise umfassen die Elektrodeneinrichtungen mindestens eine Elektrode, die derart im Bereich der Ausströmöffnung angeordnet ist, daß eine direkte Berührung des Gewebes durch die Elektrode sicher vermieden wird. Vorzugsweise zusätzlich soll auch ein Zünden eines Lichtbogens oder Plasmas in einem durch umgebende Luft verunreinigten Inertgas oder gar in Luft allein sicher vermieden werden, was (beides) die obengenannten negativen Auswirkungen mit sich brächte.

Die Elektrodeneinrichtungen umfassen mindestens eine Elektrode, die einen im wesentlichen gleichmäßigen Abstand von im wesentlichen allen Bereichen der Ausströmöffnung aufweist. Auf diese Weise ist gewährleistet, daß bei Einhaltung eines gleichmäßigen Abstandes zwischen Austrittsöffnung und Gewebe ein gleichmäßiges Plasma-Band entsteht, das wiederum eine gleichmäßige Wirkung auf das zu behandelnde Gewebe ausübt.

Die Ausströmöffnung ist vorzugsweise derart geformt und/oder angeordnet, daß der Druck des ausströmenden Inertgases über den im wesentlichen gesamten Querschnitt der Ausströmöffnung konstant ist. Dadurch wird zum einen ein besonders gleichmäßiger Gasstrom erzeugt, der durch das Fehlen von Druckunterschieden innerhalb des Gases Turbulenzen und Strahl-Verformungen vermeidet, andererseits wird sichergestellt, daß alle Bereiche des Gasstrahls "sanft" auf das Gewebe auftreffen und so eine gleichmäßige Plasma-Entstehung sichergestellt wird.

Die Ausströmöffnung ist vorzugsweise derart geformt und am Instrument angebracht, daß die Fläche des Strahls im wesentlichen senkrecht zu einer Hauptbewegungsrichtung des Instrumentes gerichtet ist. Dadurch kann eine große Läsion in einer Minimalzahl von Bewegungsabläufen behandelt werden.

Der Querschnitt der Ausströmöffnung ist vorzugsweise klein im Verhältnis zum Querschnitt einer Gaszuführungsleitung zum Leiten des Inertgases von der Gasquelle zur Ausströmöffnung. Dies führt dazu, daß die Druckabfälle innerhalb des Bereiches der Ausströmöffnung minimiert werden.
Die Ausströmöffnung ist bei einer bevorzugten Ausführungsform der Erfindung von Keramikmaterial umgeben. Besonders bei mehrfach verwendbaren Sonden stellt dies sicher, daß keine Veränderungen der Form der Austrittsöffnung beim Gebrauch auftreten.

Besonders bevorzugt ist es, das Instrument als schlauchförmige Sonde auszubilden und bei endoskopischen Operationen zu verwenden, bei welchen die Sonde durch einen Arbeitskanal eines doskops hindurchgeführt wird.

Die Ausströmöffnung ist hierbei vorzugsweise am Außenumfang der Sonde in einem Abstand zu deren distalem Ende, also gegenüber der Gasquelle angeordnet. Dieser Abstand der Ausströmöffnung zum distalen Ende der Sonde hat nicht nur strömungstechnische Vorteile, es ist vielmehr auch für die Bedienungsperson von Vorteil, wenn die Öffnung nicht ganz am distalen Ende liegt.

Bei einer Ausführungsform der Erfindung ist die Ausströmöffnung im wesentlichen parallel zur Längserstreckung der Sonde verlaufend ausgebildet. In diesem Fall wird dann mit einer drehenden Bewegung des Endoskops gearbeitet, welche die Austrittsöffnung mit ihrer Längserstreckung quer zur Bewegungsrichtung bewegt.

Bei einer anderen Ausführungsform der Erfindung ist die Ausströmöffnung im wesentlichen in Umfangsrichtung der Sonde verlaufend ausgebildet. Man kann also das Endoskop samt der Sonde an der Behandlungsstelle vorschieben und zurückziehen, so daß eine großflächige Läsion mit wenigen Bewegungen behandelt werden kann.

Bei einer alternativen Ausführungsform ist die Ausströmöffnung über einen Umfangswinkel von im wesentlichen 360° oder auch darüber schraubenförmig die Sonde umlaufend ausgebildet. Auf diese Weise ist es möglich, eine im wesentlichen gleichzeitige Behandlung eines röhrenförmigen Hohlorgans über seinen gesamten Umfang zu erzielen.
Bei der Sonde ist die Elektrode vorzugsweise drahtförmig ausgebildet, wobei der Draht mindestens im Bereich der Ausströmöffnung im wesentlichen mittig in der Sonde verläuft und an der Ausströmöffnung vorbeiführend mit einem Ende am distalen Ende der Sonde isoliert gehalten ist. Dadurch ist gewährleistet, daß die Elektrode einen gleichmäßigen Abstand von der Öffnung über deren gesamten Querschnitt aufweist. Irgendwelche gesonderten Einrichtungen, um eine Entladung zu verbessern, wie sie beispielsweise in der US 5,207,675 vorgesehen sind, sind also bei dieser Ausführungsform der Erfindung nicht vorhanden.

Die Sonde selbst weist vorzugsweise an ihrem distalen Ende, also dort, wo auch die Ausströmöffnung vorgesehen ist, ringförmige Markierungen auf, welche in gleichen Abständen voneinander vorgesehen sind. Auf diese Weise ist eine Abschätzung der Entfernungen beziehungsweise des Hervorstehens der Sonde aus dem Arbeitskanal des Endoskops im Blickfeld des Endoskops möglich. Darüber hinaus sind vorzugsweise Einrichtungen vorgesehen, um die Sonde fixiert im Arbeitskanal des Endoskops zu halten, so daß das Endoskop mit der daraus hervorstehenden Sonde ein einheitlich handhabbares Instrument bildet.

Weitere Merkmale der Erfindung ergeben sich aus den Unteransprüchen und der nachfolgenden Beschreibung bevorzugter Ausführungsformen, die anhand von Abbildungen näher erläutert werden.

Hierbei zeigen:
- Fig. 1: die perspektivische Ansicht eines Endabschnittes einer Ausführungsform einer Sonde,
- Fig. 2: eine Ansicht ähnlich der nach Fig. 1, jedoch einer anderen Ausführungsform der Sonde,
- Fig. 3: einen Längsschnitt durch die Sonde nach Fig. 1,
- Fig. 4: einen Längsschnitt durch die Sonde nach Fig. 2,
- Fig. 5: eine perspektivische Ansicht eines Endabschnittes einer Sonde gemäß einer weiteren Ausführungsform,
- Fig. 6: eine perspektivische Ansicht eines Endoskop-Endstückes mit herausragender Sonde gemäß einer weiteren Ausführungsform der Erfindung, und
- Fig. 7: ein Instrument für die offene Chirurgie gemäß einer weiteren Ausführungsform der Erfindung.

In der nachfolgenden Beschreibung werden für gleiche und gleich wirkende Teile dieselben Bezugsziffern verwendet.

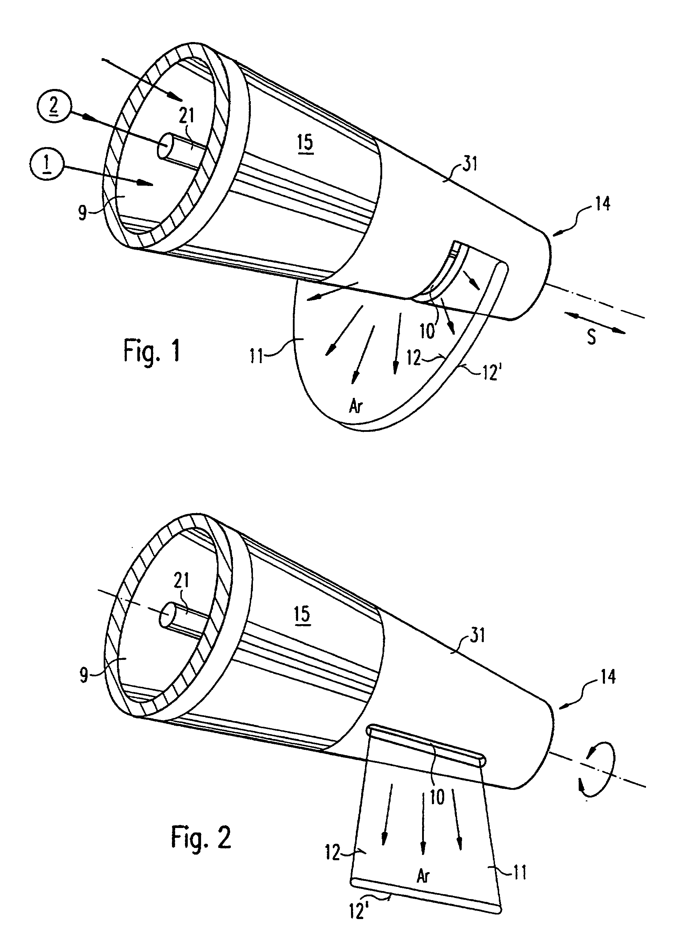

Die in Fig. 1 gezeigte Ausführungsform der Erfindung umfaßt eine schlauchförmige Sonde 31, deren Innenraum oder Lumen eine Gaszuführungsleitung 9 bildet, die mit einer Gasquelle 1 verbunden ist, welche insbesondere ein Edelgas wie Argon oder auch Helium in Richtung der gezeigten Pfeile zuführt. Im Inneren der Sonde 31 ist eine drahtförmige Stromzuführung 21 vorgesehen, die mit einer HF-Quelle 2 in an sich bekannter Weise verbunden ist.

Am Außenumfang der Sonde 31 ist ein Markierungsring 15 zu sehen, der (zusammen mit weiteren, hier nicht mehr gezeigten) Markjorungsringen dem Benutzer trotz der verzerrten Bilddarstellung im Blickfeld eines Endoskops ein Maß für das Hervorstehe der Sonde 31 aus einem Arbeitskanal des Endoskops gibt.

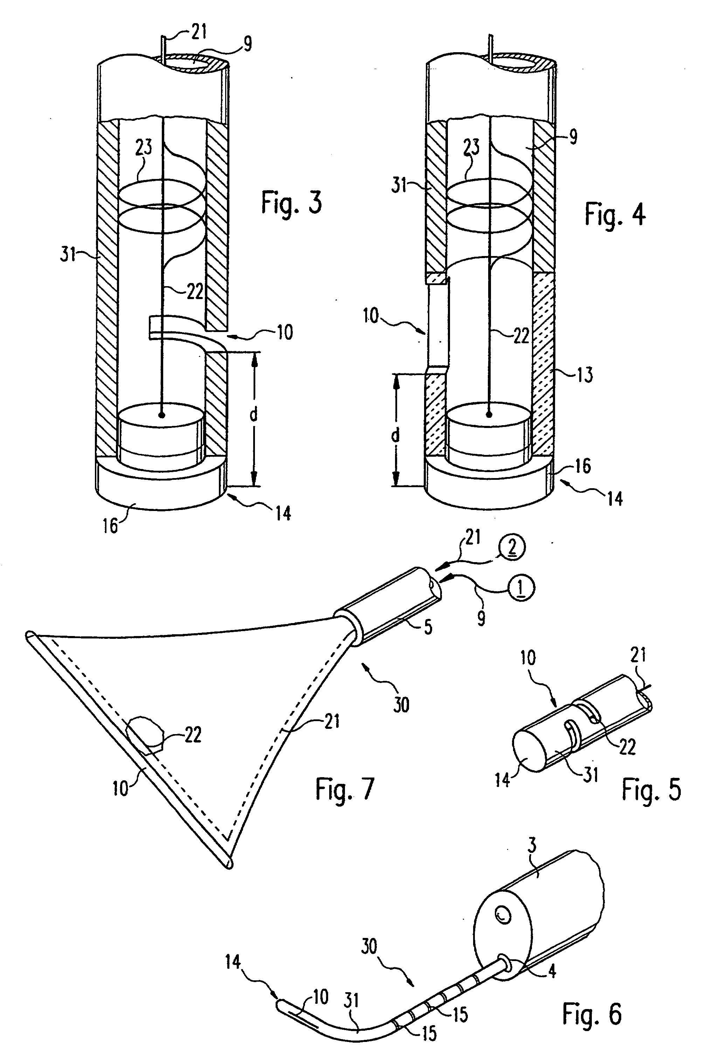

Das distale Ende 14 der Sonde 31 ist, wie dies insbesondere in Fig. 3 zu sehen ist, durch ein Endstück 16 verschlossen. In einem Abstand d vor dem distalen Ende 14 beziehungsweise der Endfläche des Endstücks 16 ist eine Ausströmöffnung 10 vorgesehen, welche die Sonde 31 bei der hier gezeigten Ausführungsform über einen Umfangswinkel von etwa 180° umläuft.
Die drahtförmige Stromzuführung 21 ist an ihrem distalen Ende, also an ihrem der HF-Quelle 2 entgegengesetzten Ende im Endstück 16 mittig gehalten. Vor der Ausströmöffnung 10 ist die drahtförmige Stromzuführung 21 durch einen Halter 23 im Zentrum der Sonde 31 gehalten, so daß zwischen dem Halter 23 und dem Endstück 16 eine im wesentlichen exakt mittig geführte Elektrode 22 entsteht, die von allen Bereichen der Ausströmöffnung 10 gleich weit entfernt ist.

Der Querschnitt der Gaszuführungsleitung 9 ist derart groß gegenüber dem Querschnitt der Ausströmöffnung 10 gewählt, daß im Bereich der Ausströmöffnung 10 überall der im wesentlichen selbe Druck herrscht. Dadurch und durch die exakte Formgebung der Randbereiche der Öffnung 10 wird gewährleistet, daß aus der Öffnung 10 ein Gasstrahl 11 austritt, der im wesentlichen die Form eines Kreisscheiben-Abschnitts aufweist, wobei Randbereiche 12, 12' des Gasstrahls 11 aufgrund der Laminarität des Gasstrahls 11 praktisch ohne Vermischung mit der umgebenden Atmosphäre ebenso wie das Innere des Strahls 11 aus reinem Gas beziehungsweise Argon bestehen. Beim Bewegen der Sonde 31 entlang des Pfeiles S (Fig. 1) kann dadurch eine großflächige Läsion in wenigen Arbeitsgängen sicher behandelt werden. Insbesondere gilt dies natürlich für Läsionen eines konkaven Gewebeabschnittes.

Die andere, in den Figuren 2 und 4 gezeigte Ausführungsform der Erfindung unterscheidet sich von der nach den Figuren 1 und 3 insbesondere durch die Formgebung und Anordnung der Ausströmöffnung 10. Bei dieser Ausführungsform ist die Ausströmöffnung 10 geradlinig, also parallel zur Achse der Sonde 31 angeordnet. Auch hier ist wieder gewährleistet, daß die Elektrode 22 einen konstanten Abstand von der Ausströmöffnung 10 aufweist. Durch diese Konstanz des Abstandes sowie durch die besondere Ausbildung des Gasstrahls, der frei von Vermischung mit umgebender Atmosphäre auf das Gewebe auftrifft, ist gewährleistet, daß nicht nur keine Berührung zwischen der Elektrode 22 und dem zu behandelnden Gewebe auftritt, es wird vielmehr auch gewährleistet, daß ein Zünden eines Plasmas in einer Gasatmosphäre, die Luft-Sauerstoff enthält, praktisch nicht auftreten kann.

Die Bewegungsrichtung der in den Figuren 2 und 4 gezeigten Sonde entspricht einer Drehung um die Längsachse der Sonde 31, wie sie in Fig. 2 mit einem Doppelpfeil angedeutet ist.

Ein weiterer Unterschied der in Fig. 4 gezeigten Ausführungsform gegenüber der nach den Figuren 1 und 3 liegt darin, daß der Endabschnitt der Sonde 31 einen Keramikabschnitt 13 umfaßt, in welchen die Ausströmöffnung 10 eingearbeitet ist und der fest mit dem schlauchförmigen Sondenabschnitt beziehungsweise der Gaszuführungsleitung 9 verbunden ist. Durch diese Ausführungsform, die natürlich in allen hier gezeigten Ausführungs formen Anwendung finden kann, ist gewährleistet, daß auch bei mehrfacher Verwendung der Sonde keine Material- und Formveränderungen durch erhöhte Temperaturen auftreten können. Darüber hinaus lassen sich mit den heute üblichen Verfahrensweisen in Keramikmaterial sehr exakte Formen, insbesondere in bezug auf die Ausströmöffnung 10 herstellen.

Die in Fig. 5 gezeigte Variante der Erfindung unterscheidet sich von den zuvor beschriebenen Ausführungsformen dadurch, daß die Ausströmöffnung 10 schraubenförmig ringsum die Sonde 31 verläuft. Dadurch wird ein Gasstrahl erzeugt, der im wesentlichen die in Fig. 1 gezeigte Form hat, jedoch ringsum die Sonde herum verläuft. Insbesondere bei relativ engen Hohlorganen ist es mit einer derartigen Sonde möglich, eine ringsum laufende Läsion in einem einzigen Arbeitsgang zu behandeln.

Bei der in Fig. 6 gezeigten Ausführungsform ist auch das Endoskop 3 mit dem Arbeitskanal 4 angedeutet. Die dort gezeigte Sonde 31, welche also ein chirurgisches Instrument 30 bildet, ist an ihrem Ende vor der Ausströmöffnung 10 gekrümmt ausgebildet. Aufgrund der Elastizität der schlauchförmigen Sonde 31, die beispielsweise aus PTFE besteht, kann man diese Sonde dennoch in den Arbeitskanal 4 einführen, wobei sie sich zunächst geradebiegt. Erst beim Austritt aus dem Arbeitskanal 4 nimmt sie wieder die in Fig. 6 gezeigte Form an. Der Grundgedanke besteht hier also darin, der Sonde eine für den besonderen Behandlungszweck optimierte Form zu geben, indem man sie entsprechend vorformt.

Bei der in Fig. 7 gezeigten Ausführungsform der Erfindung ist keine Sonde sondern ein Instrument 30 für die offene Chirurgie gezeigt. Die Ausströmöffnung 10 befindet sich hier in einem trichterförmigen Endstück, das an einem Handgriff 5 gehalten ist. Auch hier ist die Ausströmöffnung 10 wieder länglich und flach ausgebildet. Die Elektrode 22 befindet sich nach innen versetzt in einem gewissen Abstand, aber parallel zur Ausströmöffnung 10, so daß großflächige Läsionen sehr gleichmäßig behandelt werden können. Das Instrument 30 wird so geführt, daß die Ausströmöffnung 10 und damit auch die Elektrode 22 im wesentlichen parallel zum Gewebe geführt werden, das behandelt werden soll.

Bei einer anderen, hier nicht gezeigten Ausführungsform der Erfindung ist im Inneren der schlauchförmigen Sonde ein Metall-Röhrchen angebracht. Dieses dient einerseits als Elektrode, andererseits bewirkt es eine Kühlung. Das Metall-Röhrchen weist hierfür einen niedrigen Wärmewiderstand auf und ist so lang ausgebildet, daß die im Bereich der Elektrode beziehungsweise des Schlitzes entstehende Wärme durch das Gas, welches das Metall-Röhrchen durchströmt, so weit abgeführt wird, daß keine für das Kunststoffmaterial schädliche Temperatur erreicht wird.

Bei der Herstellung der Sonde geht man so vor, daß man das mit der Stromzuführung verbundene Metall-Röhrchen in die schlauchförmige Sonde einschiebt, die Sonde einspannt und die schlitzförmige Ausströmöffnung durch das Schlauchmaterial und das Röhrchen hindurch einsägt. Danach wird die Sonde durch Verschließen fertiggestellt. Auf diese Weise verläuft das Material des Röhrchens bündig mit dem Kunststoffmaterial in der Ausströmöffnung, so daß auch hier keine direkte Gewebeberührung stattfinden kann und die Gleichmäßigkeit des Abstandes von der Elektrode zur Öffnung gewährleistet ist.

### BEZUGSZEICHENLISTE

- 1: Gasquelle
- 2: HF-Quelle
- 3: Endoskop
- 4: Arbeitskanal
- 5: Handgriff
- 9: Gaszuführungsleitung
- 10: Ausströmöffnung
- 11: Strahl
- 12, 12': Randbereich
- 13: Keramikabschnitt
- 14: distales Endes
- 15: Markierungsring
- 16: Endstück
- 20: Elektrodeneinrichtung
- 21: Stromzuführung
- 22: Elektrode
- 23: Halter
- 30: Instrument
- 31: Sonde

## Patentansprüche

1. Elektrochirurgisches Instrument zur Koagulation biologischer Gewebe, umfassend
eine Gasquelle (1) zum Zuführen eines Inertgases, insbesondere eines Edelgases zu mindestens einer Ausströmöffnung (10);
eine HF-Quelle (2) zum Zuführen eines Koagulationsstroms zu einer Elektrodeneinrichtung (20), die im Bereich der mindestens einen Ausströmöffnung (10) angeordnet ist,
**dadurch gekennzeichnet, daß**
die Ausströmöffnung (10) derart schlitzförmig ausgebildet und die Gasquelle (1) derart gestaltet sind, daß das Inertgas in einem flächen- oder fächerförmigen Strahl (11) austritt, der in seinen Randbereichen (12, 12') derart laminar strömend ausgebildet ist, daß das Inertgas im wesentlichen ohne Vermischung mit umgebender Luft bis zum Gewebe gelangt.

2. Elektrochirurgisches Instrument nach Anspruch 1,
**dadurch gekennzeichnet, daß**
die Elektrodeneinrichtung (20) mindestens eine Elektrode (22) umfaßt, die derart im Bereich der Ausströmöffnung (10) angeordnet ist, daß eine direkte Berührung des Gewebes durch die Elektrode (22) sicher vermieden wird.

3. Elektrochirurgisches Instrument nach Anspruch 1,
**dadurch gekennzeichnet, daß**
die Elektrodeneinrichtung (20) mindestens eine Elektrode (22) umfaßt, die derart im Bereich der Ausströmöffnung (10) angeordnet ist, daß ein Zünden eines Lichtbogens oder Plasmas in einem umgebende Luft enthaltenden Gasgemisch oder gar in Luft allein sicher vermieden wird.

4. Elektrochirurgisches Instrument nach einem der vorhergehenden Ansprüche,
**dadurch gekennzeichnet, daß**
die Elektrodeneinrichtung (20) mindestens eine Elektrode (22) umfaßt, die einen im wesentlichen gleichmäßigen Abstand von im wesentlichen allen Bereichen der Ausströmöffnung (10) aufweist.

5. Elektrochirurgisches Instrument nach einem der vorhergehenden Ansprüche,
**dadurch gekennzeichnet, daß**
die Ausströmöffnung (10) derart geformt und/oder angeordnet ist, daß der Druck des ausströmenden Inertgases über den im wesentlichen gesamten Querschnitt der Ausströmöffnung (10) konstant ist.

6. Elektrochirurgisches Instrument nach einem der vorhergehenden Ansprüche, **dadurch gekennzeichnet, daß** die Ausströmöffnung (10) derart geformt und am Instrument (30) angebracht ist, daß die Fläche des Strahls (11) im wesentlichen senkrecht zu einer Hauptbewegungsrichtung des Instruments (30) gerichtet ist.

7. Elektrochirurgisches Instrument nach einem der vorhergehenden Ansprüche,
**dadurch gekennzeichnet, daß**
der Querschnitt der Ausströmöffnung (10) klein ist im Verhältnis zum Querschnitt einer Gaszuführungsleitung (9) zum Leiten des Inertgases von der Gasquelle (1) zur Ausströmöffnung (10).

8. Elektrochirurgisches Instrument nach einem der vorhergehenden Ansprüche,
**dadurch gekennzeichnet, daß**
die Gasausströmöffnung (10) von Keramikmaterial (13) umgeben ist.

9. Elektrochirurgisches Instrument nach einem der vorhergehenden Ansprüche,
**dadurch gekennzeichnet, daß**
das elektrochirurgische Instrument eine durch einen Arbeitskanal (4) eines Endoskops (3) hindurchführbare, im wesentlichen schlauchförmige Sonde (31) umfaßt.

10. Elektrochirurgisches Instrument nach Anspruch 9,
**dadurch gekennzeichnet, daß**
die Ausströmöffnung (10) am Außenumfang der Sonde (31) in einem Abstand (b) zu deren distalen Ende (14) gegenüber der Gasquelle (1) angeordnet ist.

11. Elektrochirurgisches Instrument nach einem der Ansprüche 9 oder 10,
**dadurch gekennzeichnet, daß**
die Ausströmöffnung (10) im wesentlichen parallel zur Längserstreckung der Sonde (31) verlaufend ausgebildet ist.

12. Elektrochirurgisches Instrument nach einem der Ansprüche 9 oder 10,
**dadurch gekennzeichnet, daß**
die Ausströmöffnung (10) im wesentlichen in Umfangsrichtung der Sonde (31) verlaufend ausgebildet ist.

13. Elektrochirurgisches Instrument nach einem der Ansprüche 9 oder 10,
**dadurch gekennzeichnet, daß**
die Ausströmöffnung (10) über einen Umfangswinkel von im wesentlichen 360° oder darüber schraubenförmig die Sonde (31) umlaufend ausgebildet ist.

14. Elektrochirurgisches Instrument nach einem der Ansprüche 9 bis 13,
**dadurch gekennzeichnet, daß**
die Elektrode (22) einen Draht umfaßt, der mindestens im Bereich der Ausströmöffnung (10) im wesentlichen mittig in der Sonde (31) gehalten ist und an der Ausströmöffnung (10) vorbeiführend mit einem Ende am distalen Ende (14) der Sonde (31) isoliert gehalten ist.

15. Elektrochirurgisches Instrument nach einem der Ansprüche 9 bis 14,
**gekennzeichnet durch**
Einrichtungen zum Fixieren der Sonde (31) im Arbeitskanal (4) derart, daß beim Koagulieren die Sonde (31) unbeweglich relativ zum distalen Ende des Endoskops (3) gehalten ist.

## Claims

1. An electrosurgical instrument for the coagulation of biological tissue, comprising
a gas source (1) for feeding an inert gas, in particular a noble gas, to at least one outflow opening (10);
an HF source (2) for feeding a coagulation current to an electrode device (20) which is disposed in the vicinity of an outflow opening (10),
**characterised in that**
the outflow opening (10) is slot-shaped and the gas source (1) is designed in such way that the inert gas emerges in a planar or fan-shaped jet (11) which in its peripheral zones (12,12') is designed to flow in a laminar stream so that the inert gas arrives at the tissue substantially without intermixing with the ambient air.

2. A surgical instrument according to Claim 1, **characterised in that** the electrode device (20) comprises at least one electrode (22) which is so arranged in the vicinity of the outflow opening (10) that any direct contact of the tissue with the electrode (22) is reliably prevented.

3. An electrosurgical instrument according to Claim 1, **characterised in that** the electrode device (20) comprises at least one electrode (22) which is so arranged in the vicinity of the outflow opening (10) that any ignition of an arc or plasma in a gas mixture containing ambient air or even in the air alone is reliably prevented.

4. An electrosurgical instrument according to any one of the preceding Claims, **characterised in that** the electrode device (22) comprises at least one electrode (22) which is at a substantially uniform distance from substantially all regions of the outflow opening (10).

5. An electrosurgical instrument according to any one of the preceding Claims, **characterised in that** the outflow opening (10) is so shaped and/or arranged that the pressure of the outflowing inert gas is constant over substantially the entire cross-section of the outflow opening (10).

6. An electrosurgical instrument according to any one of the preceding Claims, **characterised in that** the outflow opening (10) is so shaped and provided on the instrument (30) that the surface of the jet (11) is directed substantially perpendicularly to a principal direction of movement of the instrument (30).

7. An electrosurgical instrument according to any one of the preceding Claims, **characterised in that** the cross-section of the outflow opening (10) is small in relation to the cross-section of a gas-feed duct (9) for conveying the inert gas from the gas source (1) to the outflow opening (10).

8. An electrosurgical instrument according to any one of the preceding Claims, **characterised in that** the gas-outflow opening (10) is surrounded by ceramic material (13).

9. An electrosurgical instrument according to any one of the preceding Claims, **characterised in that** the electrosurgical instrument comprises a substantially hose-like probe (31) which can be passed through an operating passage (4) of an endoscope (3).

10. An electrosurgical instrument according to Claim 9, **characterised in that** the outflow opening (10) is disposed on the outer periphery of the probe (31) at a distance (b) from its distal end (14) with respect to the gas source (1).

11. An electrosurgical instrument according to either Claim 9 or Claim 10, **characterised in that** the outflow opening (10) is arranged to extend substantially parallel to the longitudinal extension of the probe (31).

12. An electrosurgical instrument according to either Claim 9 or Claim 10, **characterised in that** the outflow opening (10) is arranged to extend substantially in the circumferential direction of the probe (31).

13. An electrosurgical instrument according to either Claim 9 or Claim 10, **characterised in that** the outflow opening (10) is formed over a circumferential angle of substantially 360° or is formed to extend helically around the probe (31).

14. An electrosurgical instrument according to any one of Claims 9 to 13, **characterised in that** the electrode (22) comprises a wire which is retained, at least in the vicinity of the outflow opening (10), substantially centrally in the probe (31) and is retained insulated at one end at the distal end (14) of the probe (31) and passes by the outflow opening (10).

15. An electrosurgical instrument according to any one of Claims 9 to 14, **characterised by** means for securing the probe (31) in the operating passage (4) so that, upon coagulation, the probe (31) is retained immovably relative to the distal end of the endoscope (3).

## Revendications

1. Instrument électro-chirurgical destiné à la coagulation des tissus biologiques, comprenant
une source de gaz (1) pour l'acheminement d'un gaz inerte, en particulier un gaz rare, vers au moins un orifice de sortie (10) ;
une source de haute fréquence (2) pour l'acheminement d'un flux de coagulation vers un dispositif à électrodes (20), qui est agencé dans la zone dudit au moins un orifice de sortie (10),
**caractérisé en ce que**
l'orifice de sortie (10) est conçu en forme de fente et la source de gaz (1) est conçue de telle sorte que le gaz inerte est projeté sous la forme d'un faisceau (11) à surface plane ou en éventail, dont les zones de bordure (12, 12') sont conçues pour un écoulement laminaire de telle sorte que le gaz inerte parvient jusqu'au tissu sensiblement sans être mélangé à l'air environnant.

2. Instrument électro-chirurgical selon la revendication 1, **caractérisé en ce que** le dispositif à électrode (20) comporte au moins une électrode (22) qui est agencée dans la zone de l'orifice de sortie (10) de façon à éviter de manière sûre un contact direct du tissu avec l'électrode (22).

3. Instrument électro-chirurgical selon la revendication 1, **caractérisé en ce que** le dispositif à électrode (20) comporte au moins une électrode (22) qui est agencée dans la zone de l'orifice de sortie (10) de façon à éviter de manière sûre l'amorçage d'un arc lumineux ou d'un plasma dans un mélange gazeux contenant de l'air ambiant ou directement dans l'air.

4. Instrument électro-chirurgical selon l'une quelconque des revendications précédentes, **caractérisé en ce que** le dispositif à électrode (20) comporte au moins une électrode (22) qui est disposée à une distance sensiblement régulière de pratiquement toutes les zones de l'orifice de sortie (10).

5. Instrument électro-chirurgical selon l'une quelconque des revendications précédentes, **caractérisé en ce que** l'orifice de sortie (10) est formé et/ou agencé de telle sorte que la pression du gaz inerte sortant est constante sur pratiquement toute la section de l'orifice de sortie (10).

6. Instrument électro-chirurgical selon l'une quelconque des revendications précédentes, **caractérisé en ce que** l'orifice de sortie (10) est formé et agencé sur l'instrument (30) de telle sorte que la surface du faisceau (11) est orientée sensiblement perpendiculairement à une direction du mouvement principal de l'instrument (30).

7. Instrument électro-chirurgical selon l'une quelconque des revendications précédentes, **caractérisé en ce que** la section de l'orifice de sortie (10) est petite par rapport à la section d'une conduite d'acheminement de gaz (9) destinée à guider le gaz inerte à partir de la source de gaz (1) vers l'orifice de sortie (10).

8. Instrument électro-chirurgical selon l'une quelconque des revendications précédentes, **caractérisé en ce que** l'orifice de sortie du gaz (10) est entouré par un matériau céramique (13).

9. Instrument électro-chirurgical selon l'une quelconque des revendications précédentes, **caractérisé en ce que** l'instrument électro-chirurgical comporte une sonde (31) sensiblement en forme de tuyau flexible, destinée à passer dans un canal de travail (4) d'un endoscope (3).

10. Instrument électro-chirurgical selon la revendication 9, **caractérisé en ce que** l'orifice de sortie (10) est agencé sur le pourtour extérieur de la sonde (31) à une distance (b) de l'extrémité distale (14) de celle-ci par rapport à la source de gaz (1).

11. Instrument électro-chirurgical selon la revendication 9 ou 10, **caractérisé en ce que** l'orifice de sortie (10) est conçu pour être orienté sensiblement parallèlement à la dimension longitudinale de la sonde (31).

12. Instrument électro-chirurgical selon la revendication 9 ou 10, **caractérisé en ce que** l'orifice de sortie (10) est conçu pour s'étendre sensiblement dans le sens périphérique de la sonde (31).

13. Instrument électro-chirurgical selon la revendication 9 ou 10, **caractérisé en ce que** l'orifice de sortie (10) est conçu pour entourer la sonde (31) sur un angle circulaire de sensiblement 360° ou en forme de spirale sur celle-ci.

14. Instrument électro-chirurgical selon l'une quelconque des revendications 9 à 13, **caractérisé en ce que** l'électrode (22) comporte un fil qui, au moins dans la zone de l'orifice de sortie (10), est maintenu sensiblement au centre de la sonde (31), passe devant l'orifice de sortie (10) et est maintenu de manière isolée avec une extrémité au niveau de l'extrémité distale (14) de la sonde (31).

15. Instrument électro-chirurgical selon l'une quelconque des revendications 9 à 14, **caractérisé par** des dispositifs destinés à bloquer la sonde (31) dans le canal de travail (4), de telle sorte que, au moment de la coagulation, la sonde (31) est immobilisée par rapport à l'extrémité distale de l'endoscope (3).
